# EUROPEAN PATENT SPECIFICATION

(11) **EP 0 610 423 B1**
(45) Date of publication and mention of the grant of the patent: **07.05.1997**
(21) Application number: 92924131.3
(22) Date of filing: 28.10.1992
(51) Int. Cl.: A61L 27/00, C12N 5/08

(54) **PREVASCULARIZED POLYMERIC IMPLANTS FOR ORGAN TRANSPLANTATION**
VORVASKULARISIERTE POLYMERIMPLANTATE FÜR ORGANTRANSPLANTATION
IMPLANTS POLYMERES PREVASCULARISES POUR LA TRANSPLANTATION D'ORGANES

(30) Priority: 30.10.1991 US 785021
(43) Date of publication of application: 17.08.1994
(73) Proprietor: MASSACHUSETTS INSTITUTE OF TECHNOLOGY, Cambridge, MA 02139 (US); CHILDREN'S MEDICAL CENTER CORPORATION, Boston Massachusetts 02115 (US)
(72) Inventor: STEIN, James, E., Boston, Massachusetts 02116-3952 (US); GILBERT, James, C., Decatur, GA 30033 (US); INGBER, Donald, E., Boston, MA 02116 (US); LANGER, Robert, S., Newton, MA 02158 (US); VACANTI, Joseph, P., Winchester, MA 01890 (US)
(74) Representative: Bassett, Richard Simon
(86) International application number: US9209142
(87) International publication number: WO9308850

(56) References cited:
- WO-A-88/03785
- WO-A-89/07944
- WO-A-90/12603
- WO-A-90/12604

## Description

### Background of the Invention

This invention is generally in the field of medicine and cell culture, and in particular in the area of implantable organs formed on biocompatible artificial matrices.

Loss of organ function can result from congenital defects, injury or disease. Many times treatment with drugs or surgery is not in itself sufficient and the patient dies or is severely disabled. One approach for treatment has been to transplant donor organs or tissue into the patient. Drugs such as cyclosporin can be used to prevent tissue rejection. However, there is a tremendous shortage of donor organs, most of which must come from a recently deceased individual.

There have been a number of attempts to culture dissociated tissue and implant the cells directly into the body. For example, transplantation of pancreatic tissue, either as a whole organ or as a segment of an organ, into the diabetic patient has been attempted. Serum glucose appears to be controlled in a more physiological manner using this technique and the progression of complications is thereby slowed. An earlier approach which was not successful in achieving long-term benefits was the transplantation of islet cells through injection of isolated clusters of islet cells into the portal circulation, with implantation in the vascular bed of the liver. More recent methods have included encapsulation of pancreatic beta cells to prevent immune attack by the host and injection of fetal beta cells beneath the capsule of the kidney. Although there is evidence of short term function, long term results have been less satisfactory (D.E.R. Sutherland, Diabetologia 20, 161-185 (1981); D.E.R. Sutherland, Diabetologia 20,435-500 (1981)). Currently whole organ pancreatic transplantation is the preferred treatment.

One of the problems with implanting dissociated cells into the body is that they do not form three dimensional structures and the cells are lost by phagocytosis and attrition. One approach to overcome this problem is described by U.S. Patent No. to Lim, wherein cells are encapsulated within spheres, then implanted. While this method can sometimes maintain viable functioning cells, the cells do not form organs or structures and rarely result in long term survival and replication of the encapsulated cells. Most cells have a requirement for attachment to a surface in order to replicate and to function.

The first attempts to culture cells on a matrix for use as artificial skin, which requires formation of a thin three dimensional structure, were described by Yannas and Bell in a series of publications. They used collagen type structures which were seeded with cells, then placed over the denuded area. A problem with the use of the collagen matrices was that the rate of degradation is not well controlled. Another problem was that cells implanted into the interior of thick pieces of the collagen matrix failed to survive.

One method for forming artificial skin by seeding a fibrous lattice with epidermal cells is described in U.S. Patent No. 4,485,097 to Bell, which discloses a hydrated collagen lattice that, in combination with contractile agents such as platelets and fibroblasts and cells such as keratinocytes, is used to produce a skin-equivalent. U.S. Patent No. 4,060,081, to Yannas et al. discloses a multilayer membrane useful as synthetic skin which is formed from an insoluble non-immunogenic material which is nondegradable in the presence of body fluids and enzymes, such as cross-linked composites of collagen and a mucopolysaccharide, overlaid with a non-toxic material such as a synthetic polymer for controlling the moisture flux of the overall membrane. U.S. Patent No. 4,458,678 to Yannas et al. discloses a process for making a skin-equivalent material wherein a fibrous lattice formed from collagen cross-linked with glycosaminoglycan is seeded with epidermal cells.

A disadvantage to the first two methods is that the matrix is formed of a "permanent" synthetic polymer. The '678 patent has a feature that neither of the two prior patents has, a biodegradable matrix which can be formed of any shape, using the appropriate cells to produce an organ such as the skin. Unfortunately, there is a lack of control over the composition and configuration of the latter matrices since they are primarily based on collagen. Further, since collagen is degraded by enzymatic action as well as over time by hydrolysis, the degradation is quite variable.

U.S. Patent No. 4,520,821 to Schmidt describes a similar approach that was used to make linings to repair defects in the urinary tract. Epithelial cells were implanted onto synthetic matrices, where they formed a new tubular lining as the matrix degraded. The matrix served a two fold purpose - to retain liquid while the cells replicated, and to hold and guide the cells as they replicated.

In EP 0 299 010 entitled "Chimeric Neomorphogenesis of Organs Using Artificial Matrices" filed November 20, 1986 by Joseph P. Vacanti and Robert S. Langer, a method of culturing dissociated cells on biocompatible, biodegradable matrices for subsequent implantation into the body was described. This method was designed to overcome a major problem with previous attempts to culture cells to form three dimensional structures having a diameter of greater than that of skin. Vacanti and Langer recognized that there was a need to have two elements in any matrix used to form organs: adequate structure and surface area to implant a large volume of cells into the body to replace lost function and a matrix formed in a way that allowed adequate diffusion of gases and nutrients throughout the matrix as the cells attached and grew to maintain viability in the absence of vascularization. Once implanted and vascularized, the porosity required for diffusion of the nutrients and gases was no longer critical.

However, even with the method described by Vacanti, the implant was initially constructed *in vitro*, then implanted. It is clearly desirable to be able to avoid the *in vitro* step. EP 0 422 209 by Vacanti, et al., describes an approach used to address this problem. Recognizing the need for vascularization to maintain the implant *in vitro*, first addressed in the 1986 patent application by Vacanti, et al., the implant was seeded *in vitro* then immediately implanted into a highly vascularized tissue, the mesentery. A drawback with this was that the implant could only be made into this area of the body, and that a number of thin implants had to be used to achieve the requisite number of cells.

It is therefore an object of the present invention to provide an implant containing the requisite number of cells to replace lost organ function.

It is a further object of the present invention to provide a biocompatible, polymeric implant which can be implanted with cells without prior *in vitro* culturing and then degrades at a controlled rate over a period of time as the implanted cells replicate and form an organ structure.

### Summary of the Invention

A method is disclosed whereby cells having a desired function are seeded on and into biocompatible, biodegradable or non-degradable polymer scaffolding, previously implanted in a patient and infiltrated with blood vessels and connective tissue, to produce a functional organ equivalent. The resulting organoid is a chimera formed of parenchymal elements of the donated tissue and vascular and matrix elements of the host.

The matrix should be a pliable, non-toxic, injectable porous template for vascular ingrowth. The pore size, usually between approximately 100 and 300 microns, should allow vascular and connective tissue ingrowth throughout approximately 10 to 90% of the matrix, and the injection of cells such as hepatocytes without damage to the cells or patient. The introduced cells attach to the connective tissue and are fed by the blood vessels. The preferred material for forming the matrix or support structure is a biodegradable synthetic polymer, for example, polyglycolic acid, polylactic acid, polyorthoester, polyanhydride, or copolymers thereof, or a sponge derivatized from polyvinyl alcohol. The elements of these materials can be overlaid with a second material to enhance cell attachment. The polymer matrix must be configured to provide access to ingrowing tissues to form adequate sites for attachment of the required number of cells for viability and function and to allow vascularization and diffusion of nutrients to maintain the cells initially implanted. An advantage of the biodegradable material is that compounds such as angiogenic factors, biologically active compounds which enhance or allow ingrowth of the blood vessels, and lymphatic network or nerve fibers, may be incorporated into the matrix for slow release during degradation of the matrix.

Cells of one or more types can be selected and grown on the matrix. A preferred type of cell is a parenchymal cell such as a hepatocyte, which is difficult to culture under normal conditions. Cells genetically engineered to include genes encoding proteins which would otherwise be absent, such as those resulting from liver protein deficiencies and metabolic defects such as cystic fibrosis, can be implanted with this method.

In the preferred embodiment for implanting cells with a high oxygen requirement such as hepatocytes, the porous implant containing an indwelling catheter is implanted into the mesentery, prevascularized for a period of time, such as five days, and cells injected. In the most preferred embodiment for hepatocytes, immediately prior to polymer implantation portacaval shunts are created to provide trophic stimulatory factors to the implants to enhance replication and function.

### Brief Description of the Drawings

Figure 1 is the tissue ingrowth (µ) into Ivalon, at four days, five days, and six days.

Figures 2a and 2b are the vascularity of Ivalon over time, vessels/HPF (Figure 3a) and vessel area (µ²) (Figure 3b) at four days, five days, six days, and fourteen days.

Figure 3 is a graph of the effect of hepatotrophic stimulation on implant growth, cell area (µm²) for control, 70% hepatectomy alone, and portacaval shunt in combination with 70% hepatectomy.

Figure 4 is a graph of hepatocyte survival in Ivalon, hepatocyte area (µ²) at 0 time, 24 hours, and one week for 5 million cells and 10 million cells.

### Detailed Description of the Invention

Disclosed herein is a method to provide functional organ equivalents using artificial substrates as scaffolding for cellular transfer and implantation. Cell shape is determined by cytoskeletal components and attachment to matrix plays an important role in cell division and differentiated function. If dissociated cells are placed into mature tissue as a suspension without cell attachment, they may have difficultly finding attachment sites, achieving polarity, and functioning because they begin without intrinsic organization. This limits the total number of implanted cells which can remain viable to organize, proliferate, and function.

For an organ to be constructed, successfully implanted, and function, the matrices must have sufficient surface area and exposure to nutrients such that cellular growth and differentiation can occur prior to the ingrowth of blood vessels following implantation. The time required for successful implantation and growth of the cells within the matrix is greatly reduced if the area into which the matrix is implanted is prevascularized. After implantation, the configuration must allow for diffusion of nutrients and waste products and for continued blood vessel ingrowth as cell proliferation occurs.

Cells can either be implanted after seeding onto a matrix or injected into a matrix already implanted at the desired site. The latter has the advantage that the matrix can be used to prevascularize the site.

### Design and construction of scaffolding:

The design and construction of the scaffolding is of primary importance. The matrix should be a pliable, non-toxic, injectable porous template for vascular ingrowth. The pores should allow vascular ingrowth and the injection of cells such as hepatocytes without damage to the cells or patient. These are generally interconnected pores in the range of between approximately 100 and 300 microns. The matrix should be shaped to maximize surface area, to allow adequate diffusion of nutrients and growth factors to the cells and to allow the ingrowth of new blood vessels and connective tissue. At the present time, a porous structure that is resistant to compression and/or tension is preferred.

In the preferred embodiment, the matrix is formed of a bioabsorbable, or biodegradable, synthetic polymer such as a polyanhydride, polyorthoester, polylactic acid, polyglycolic acid, and copolymers or blends thereof. Non-degradable materials can also be used to form the matrix. Examples of suitable materials include ethylene vinyl acetate, derivatives of polyvinyl alcohol, polytetrafluoroethylene (teflon®), and nylon. The preferred non-degradable materials are a polyvinyl alcohol sponge, or alkylation, and acylation derivatives thereof, including esters. A non-absorbable polyvinyl alcohol sponge is available commercially as Ivalon™, from Unipoint Industries. Methods for making this material are described in U.S. Patent Nos. 2,609,347 to Wilson; 2,653,917 to Hammon, 2,659,935 to Hammon, 2,664,366 to Wilson, 2,664,367 to Wilson, and 2,846,407 to Wilson, the teachings of which are incorporated by reference herein. Collagen can be used, but is not as controllable and is not preferred. These materials are all commercially available. Non-biodegradable polymer materials can be used, depending on the ultimate disposition of the growing cells, including polymethacrylate and silicon polymers.

In some embodiments, attachment of the cells to the polymer is enhanced by coating the polymers with compounds such as basement membrane components, agar, agarose, gelatin, gum arabic, collagens types I, II, III, IV, and V, fibronectin, laminin, glycosaminoglycans, mixtures thereof, and other materials known to those skilled in the art of cell culture.

All polymers for use in the matrix must meet the mechanical and biochemical parameters necessary to provide adequate support for the cells with subsequent growth and proliferation. The polymers can be characterized with respect to mechanical properties such as tensile strength using an Instron tester, for polymer molecular weight by gel permeation chromatography (GPC), glass transition temperature by differential scanning calorimetry (DSC) and bond structure by infrared (IR) spectroscopy, with respect to toxicology by initial screening tests involving Ames assays and *in vitro* teratogenicity assays, and implantation studies in animals for immunogenicity, inflammation, release and degradation studies.

In a preferred embodiment, the matrix contains catheters for injection of the cells into the interior of the matrix after implantation and ingrowth of vascular and connective tissue. Catheters formed of medical grade silastic tubing of different diameters and of differing exit ports to allow even distribution of cells throughout the matrix, as described in the following examples, are particularly useful. Other methods can also be used, such as molding into the matrix distribution channels from the exterior into various parts of the interior of the matrix, or direct injection of cells through needles into interconnected pores within the matrix.

### Preparation of Cells for implantation:

Cells can be obtained directly from a donor organ, from cell culture of cells from a donor organ, or from established cell culture lines. In the preferred embodiments, cells are obtained directly from a donor organ, washed and implanted directly into a pre-implanted, pre-vascularized matrix. The cells are cultured using techniques known to those skilled in the art of tissue culture.

In one variation of the method using a single matrix for attachment of one or more cell lines, the scaffolding is constructed such that initial cell attachment and growth occur separately within the matrix for each population. Alternatively, a unitary scaffolding may be formed of different materials to optimize attachment of various types of cells at specific locations. Attachment is a function of both the type of cell and matrix composition. Cell attachment and viability can be assessed using scanning electron microscopy, histology, and quantitative assessment with radioisotopes.

Although the presently preferred embodiment is to utilize a single matrix implanted into a host, there are situations where it may be desirable to use more than one matrix, each implanted at the most optimum time for growth of the attached cells to form a functioning three-dimensional organ structure from the different matrices.

The function of the implanted cells, both *in vitro* as well as *in vivo*, must be determined. *In vivo* liver function studies can be performed by placing a cannula into the recipient's common bile duct. Bile can then be collected in increments. Bile pigments can be analyzed by high pressure liquid chromatography looking for underivatized tetrapyrroles or by thin layer chromatography after being converted to azodipyrroles by reaction with diazotized azodipyrroles ethylanthranilate either with or without treatment with P-glucuronidase. Diconjugated and monoconjugated bilirubin can also be determined by thin layer chromatography after alkalinemethanolysis of conjugated bile pigments. In general, as the number of functioning transplanted hepatocytes increases, the levels of conjugated bilirubin will increase. Simple liver function tests can also be done on blood samples, such as albumin production. Analogous organ function studies can be conducted using techniques known to those skilled in the art, as required to determine the extent of cell function after implantation. Studies using labelled glucose as well as studies using protein assays can be performed to quantitate cell mass on the polymer scaffolds. These studies of cell mass can then be correlated with cell functional studies to determine what the appropriate cell mass is.

### Methods of Implantation

The technique described herein can be used for delivery of many different cell types to achieve different tissue structures. For example, islet cells of the pancrease may be delivered in a similar fashion to that specifically used to implant hepatocytes, to achieve glucose regulation by appropriate secretion of insulin to cure diabetes. Other endocrine tissues can also be implanted. The matrix may be implanted in many different areas of the body to suit a particular application. Sites other than the mesentery for hepatocyte injection in implantation include subcutaneous tissue, retroperitoneum, properitoneal space, and intramuscular space.

Implantation into these sites may also be accompanied by portacaval shunting and hepatectomy, using standard surgical procedures. The need for these additional procedures depends on the particular clinical situation in which hepatocyte delivery is necessary. For example, if signals to activate regeneration of hepatocytes are occurring in the patient from his underlying liver disease, no hepatectomy would be necessary. Similarly, if there is significant portosystemic shunting through collateral channels as part of liver disease, no portacaval shunt would be necessary to stimulate regeneration of the graft. In most other applications, there would be no need for portacaval shunting or hepatectomy.

The methods using polymeric implants and pre-vascularization, as described above, will be further understood by reference to the following examples.

### Example 1: Determination of factors required for in vivo survival of cells.

Methods described in prior patent applications were used to determine the relative importance of various factors in survival of the cells following implantation. Hepatocytes were studied immediately after isolation, when placed on polymer constructs *in vitro*, and at various time points starting at time zero after implantation. Standard hepatocyte isolation techniques were used. Implantation consisted of attaching cells of varying densities to a polymer fiber complex *in vitro* and then implantation of this complex. The intestinal mesentery was the implantation site.

The results demonstrated that it was possible to achieve consistently high viabilities of well functioning hepatocytes pre-implantation. Viability ranged from 85-90%. Routine Percoll separation was found to improve viability. It was also determined that the viability and function remained high on the polymer constructs *in vitro*. By contrast, using the prior art method, there was immediate massive loss of cell viability and function after implantation. Morphometric approaches to quantitative analysis of these fiber complexes were performed but were difficult since viable cells were so rare in the graft. Quantitative estimates of cell loss were between 95-97%. Histologically, there seemed to be stability of the remaining cell masses after the first 24-28 hours with long term engraftment and function of these cells out to one year as documented by *in situ* histochemical staining for albumin. The hepatocytes remained in clusters varying between 100-250 microns in diameter. These clusters were consistent in appearance independent of cell density application and location of implantation site. The clusters always were predominant in regions closest to the native tissue and blood vessels. These observations suggested that diffusion limitations, especially of oxygen, were the most likely contributors of hepatocyte death. In contrast, similar experiments using chondrocytes to make new cartilage were highly successful with formation of homogeneous plates of cartilage, again suggesting that the particular sensitivity of the hepatocyte to hypoxic damage was the problem.

### Example 2: Prevascularization and implantation of cells on polylactic acid and polyglycolic acid matrices.

### Design of Polymers

Silastic tubing (.3 mm ID) was divided into 2.5 inch lengths. One end was scaled and .25 mm holes cut into the tubing. These injection catheters were introduced centrally into 1 cm discs of polyvinyl alcohol foam (Ivalon, Unipoint Indust.) with a 5 mm thickness. These devices were then sterilized for implantation.

### Animal Implantation

200-250 g Fisher 344 rats were anaesthetized with methoxyflurane and the abdomen prepped. A midline incision was made and the mesentery carefully laid out on sterile gauze. The polymer was then placed onto the mesentery which was then folded back on the device to encase it. The polymer was fixed in place with a single 6-0 Prolene™ (Ethicon) suture. At this point an incision was made in the lateral abdominal wall and the injection catheter led through the muscle to a subcutaneous pocket and fixed. The abdomen was closed and skin approximated. This could then be accessed later for atraumatic introduction of hepatocytes.

### Histologic Examination

After appropriate formalin fixation, each prevascularized implant was sectioned at four predetermined sites perpendicular to the axis of the injection catheter. Hematoxylin and Eosin (H&E) staining was performed and the sections evaluated for vascularity, tissue ingrowth and viable hepatocyte area. Quantification of these parameters was carried out using a model 3000 Image Analyser.

### Hepatocyte isolation

Hepatocytes were isolated using a modification of the Seglan technique [Aiken, 1990 #13]. A syngeneic donor Fisher 344, 200-250 g rat was anaesthetized with methoxyflurane. The liver was exposed and the IVC cannulated with a 16 g angiocath. A 6 minute perfusion with calcium free buffered perfusate at 38°C was carried out. This was followed by perfusion with .05% Collagenase D (Boehringer Mannheim) in a 0.05 M calcium chloride containing buffer until adequate hepatocyte dissociation was achieved. The hepatocytes were then purified using Percoll density centrifugation. Viability was determined by Trypan Blue nuclear exclusion.

### Hepatocyte Injection

Hepatocytes were suspended in Williams E medium (Gibco) at 1 x 10⁷ and 2 x 10⁷ viable cells/cc. Following 5 days of polymer prevascularization, rats receiving cell injections were anaesthetized, the subcutaneous injection catheter exposed and 0.5 cc of cell suspension was injected and animals harvested either immediately after injection, 1 day after injection or 7 days following injection. Catheters were replaced in the subcutaneous pocket and skin reapproximated.

### Results

### Tissue Ingrowth

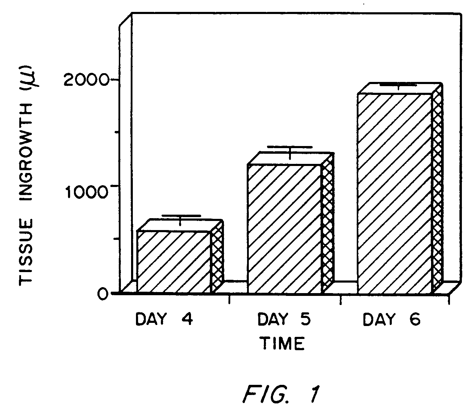

Devices were harvested following 1 to 14 days of prevascularization. Tissue ingrowth occurred at a very consistent rate over time. Between day 1 and day 3 fibrin clot deposition with increasing cellularity was noted. There was no evidence of tissue organization or vascular ingrowth noted during this time. At day 4 organized tissue,as well as capillaries, could be noted extending into the interconnected interstices of the device. Tissue ingrowth was symmetric from both sides of the device until confluence was reached at day 7 of prevascularization. The rate of tissue ingrowth was constant at 604 µm/day (range 575-627 µm/day) between days 4 and 7, as shown in Figure 1.

It appears to be essential that spaces are consistently maintained between the polymer and the tissue. It is this space which crate channels for the injection and implantation of hepatocytes.

### Vascularity

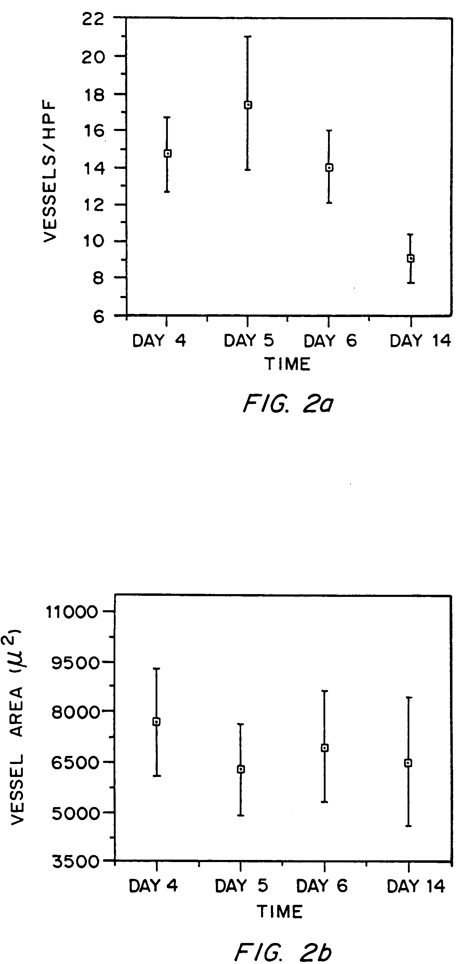

Vascularity was assessed in two ways. First, the vessel number per unit area was determined at multiple preselected sites within the polymer and an average obtained. Second, the vessel area within these same fields was determined. The field which was quantified was the most central extent of organized tissue ingrowth. The results are depicted graphically in Figures 2a and b. Once tissue organization occurred, vessel number/HPF reached a maximal density of 17 at day 5 of prevascularization before declining to 9 vessels/HPF by day 14. This was in contrast to vessel area which remained constant over time, indicating a progression from smaller to larger vessels over this period.

### Hepatocyte Distribution and Survival

The hepatocytes distributed themselves evenly throughout the polymer at the time of cell injection. Viable cells became increasingly localized to the interface of the mesentery and polymer over time. Histologically it was demonstrated that the cells initially viable require attachment to the fibrovascular tissue network to survive. Those not in contact become nonviable within 24 hours. By one week hepatocytes were limited to the outer edge of the device. The cells did not appear to thrive in the central portion of the polymer even following attachment to the tissue ingrowth. Remodeling occurred and the cells which did engraft became incorporated into the fibrovascular tissue as islands of 4-5 cells or as a 2-3 cell layer sheet around the outer margins of the polymer. Hepatocyte area within the polymer was examined to assess survival. A 40% decrease in viable hepatocyte area over the first 24 hours with a gradual decrease to 25% of initial hepatocyte area by 1 week was shown. Examination of implants at 4 months demonstrated a continued fall in hepatocyte area to between 5 and 10% of cells implanted at time 0. Increasing the number of cells injected by 100% provided a 100% increase in viable hepatocyte area with a proportional decrease in area over time.

### Example 3: Implantation of hepatocytes using porous polyvinyl alcohol implants.

A number of studies, including example 1, indicated there was a massive loss of hepatocytes using the technique of attaching hepatocytes to polymer fibers of polyglycolic aid in cell culture and then implanting these polymer cell constructs into the mesentery of the intestine. To approach these problems, new assays to assess cell viability and function before, during, and after cell implantation, alternative cell isolation techniques, new materials to improve cell viability, and systems of prevascularization to improve vascularized surface area for implantation were developed. As a result of these efforts, it was determined that the major cause of cell death was related to variables at the time of implantation. Most cell death occurred within the first six hours after implantation. 95-97% of hepatocytes were lost in this early period after implantation.

Morphometric techniques to analyze tissue sections of the implants *in vivo* were developed. This analysis *in vivo* was coupled with the development of quantitative "Northern" blot analysis to measure total RNA as well as liver-specific albumin mRNA within the implant. These *in vivo* observations could be compared to *in vitro* RNA analysis of cells on polymer as well as measurement of albumin production using gel electrophoresis. *In vitro* and *in vivo* analysis measuring viability using MTT(30)4, 5-dimethyl thiazol-2-yl(-2, 5-diphenyl tetrazolium bromide), was used in the assay. This biochemical assay was used as a non specific marker of cell viability and was compared to acid phosphatase measurements.

A system utilizing porous polymers of polyvinyl alcohol allowing successful transplantation, defined as long term engraftment and organization of hepatocytes and biliary duct-like structures, was developed. Cell survival was increased to the 60-70% range by developing polymer systems allowing prevascularization into sponge like porous materials. The efficiency of delivery of cells is estimated to be between 40 and 60% at 24 to 28 hours after implantation. The results indicate that the strategy of prevascularization into a sponge like geometry with secondary introduction of hepatocytes significantly decreases early cell loss.

Gunn rats (150-250 g) were anaesthetized and end to side portacaval shunts created. 1.5 x 1.5 cm polymer sponges with central multiport silastic tubes for cell injection were placed in mesenteric envelopes or subcutaneous pockets (n=20). After 5 days of prevascularization, hepatocytes were isolated from a syngeneic Wistar rat by collagenase perfusion. 1 x 10⁷ hepatocytes were injected. Three days after engraftment a 70% hepatectomy was performed and the implants serially evaluated by H&E sectioning out to six months. Individual cross-sectional areas of ductal structures in native liver, heterotopic transplanted grafts and the implants were compared by morphometric quantification. Analysis of variance was used to assess statistical significance.

Hepatocyte engraftment and reorganization occurred in all implants out through six months. Organized nodules of up to 1 mm were identified with hepatocytes arranged in plates. 30% of the implants at 4 and 6 months (n=10) contained tubular structures lined by cuboidal epithelium with a histologic appearance similar to those in heterotopic transplants with bile duct hyperplasia. The area of these ducts was compared to interlobular ducts, the smallest biliary structures with cuboidal epithelium, in heterotopic grafts and native liver. The ducts in the implants had a mean area of 745 µ² ± 47, the hyperplastic ducts within the heterotopic transplant, 815 µ² ± 41 (p = 0.34), while those with comparable morphology from the portal triad of native liver had a mean of 1360 µ² ± 105 (p<.001).

Both long term engraftment and development of ductal structures within organized nodules of hepatic tissue in both mesenteric and subcutaneous hepatocyte implants was demonstrated. These structures, which are morphologically and morphometrically similar to those seen in bile duct hyperplasia in heterotopic liver transplants, represent the first evidence suggestive of bile duct organization following hepatocyte transplantation.

### Example 4: Comparison of polymer fiber matrices with polyvinyl alcohol sponge matrices.

Empty polymers were implanted to allow fibrovascular ingrowth into the complex before hepatocyte introduction to increase vascularized surface area, thereby allowing shorter diffusion distances for oxygen delivery. Several geometric configurations were tested, including 1) bioabsorbable polymer fibers only, 2) nondegradable polymer fibers, 3) mixtures of degradable as well as nondegradable fibers, 4) cellulose sponges, 5) Ivalon sponges.

All of the unsupported fiber complexes were unsuitable for prevascularization for two reasons. First of all, they did not have enough resistance to compression and thus contraction occurred as fibrovascular tissue migrated. This also created a very high resistance to introduction of hepatocytes, whether they were introduced by direct injection or by an indwelling multiport catheter. Direct injection produced bleeding within the interstices of the implant.

A sponge model was then developed since it seemed to have greater resistance to compression and allowed for maintenance of potential spaces. Many studies were performed with both Ivalon sponges and the cellulose sponge to determine the time course for vasculazation. Good vascular ingrowth occurred in both models. The standard Ivalon sponge implant was a disc of one centimeter diameter by 0.3 cm in height. There was good vascular ingrowth by day 5 and very thorough vascular ingrowth by day 11. The pores were of quite uniform size and all interconnected. A system was designed in which a central multiport catheter was placed into the Ivalon disc so that hepatocytes could be introduced either as a single injection or multiple injections. Although the cellulose sponge allowed for good vascular ingrowth with minimal inflammation, the pores were of very inconsistent size and therefore made it less suitable than the Ivalon.

### Methods

### Prevascularized Ivalon Sponge

Ivalon sponges were placed into the mesentery of Fischer 344 rats and allowed to prevascularize for varying numbers of days. At designated times hepatocytes were injected through the centrally placed silastic catheter. The concentration of hepatocytes and final volume infused into the sponges were varied. The animals were perfused with formalin at Time 0 and three days after hepatocyte injection. This was chosen based on prior work which has shown fairly consistent cell survival after this point and that the hepatocyte loss occurred over the first 24 hours. The cell/polymer constructs were then sectioned and evaluated for tissue ingrowth, vascularity, hepatocyte distribution and viability. Quantitation of the viable hepatocytes was carried out using morphometric image analysis.

To determine cell number, each implant was divided into four pieces and a section made from each of these. Each of these sections was then examined through three cross sections microscopically and cell area determined. Average cell area and volume were then determined and cell number extrapolated for the volume of the sponge.

### RNA Isolation from Polymer Implants

Hepatocytes were isolated by collagenase perfusion and centrifugation through Percoll. Prior to injection into prevascularized Ivalon sponge, cellular RNA was labeled in suspension with 10 µCi/ml³H-uridine. Pre-labeling hepatocyte RNA allows distinction between hepatocyte RNA and that of infiltrating cells. 5x10⁶ labeled hepatocytes were then injected into Ivalon sponge as described elsewhere. In previous experiments using polyglycolic acid (PGA), cells were applied to polymer in a tissue culture dish, incubated overnight in 10 µCi/ml³H-uridine, and implanted as previously described.

To isolate RNA, implants were removed at time intervals and placed in a guanidinium isothiocyanate lysis solution. Multiple aliquots of this solution were worked through the sponge and combined. The RNA was purified by subsequent phenol-chloroform extractions, and lithium chloride and ethanol precipitations. The final RNA samples were applied to a nitrocellulose filter using a slot blot apparatus (Schleicher and Schuell). Following hybridization with a ³²P-labeled cDNA probe specific for albumin mRNA, the filter was washed and placed on X-ray film. The resulting autoradiograph was scanned with a densitometer to quantitate relative amounts of albumin mRNA in each sample. Alternatively, RNA samples can be electrophoresed to separate mRNA species, blotted onto nitrocellulose, and probed as usual. This latter procedure is termed "Northern" blot analysis.

### Results

### Prevascularized Ivalon Sponge

### Tissue ingrowth

Infiltrating tissue completely bridged the 3 mm thickness of Ivalon in 7 days. The extent of its fibrous and vascular components increase over time. Highly vascular, minimally fibrous tissue was present at 5 days. At this time there were still relatively hypocellular areas centrally in the implant. Tissue became further organized through day 14 with a decrease in potential space for cell implantation.

### Distribution of cells with injection

Cell distribution remained consistent over the entire time course with an even distribution of cells immediately after injection. Over the three days following injection, cell survival became more predominant at the periphery where the new tissue was more organized.

### Hepatocyte survival

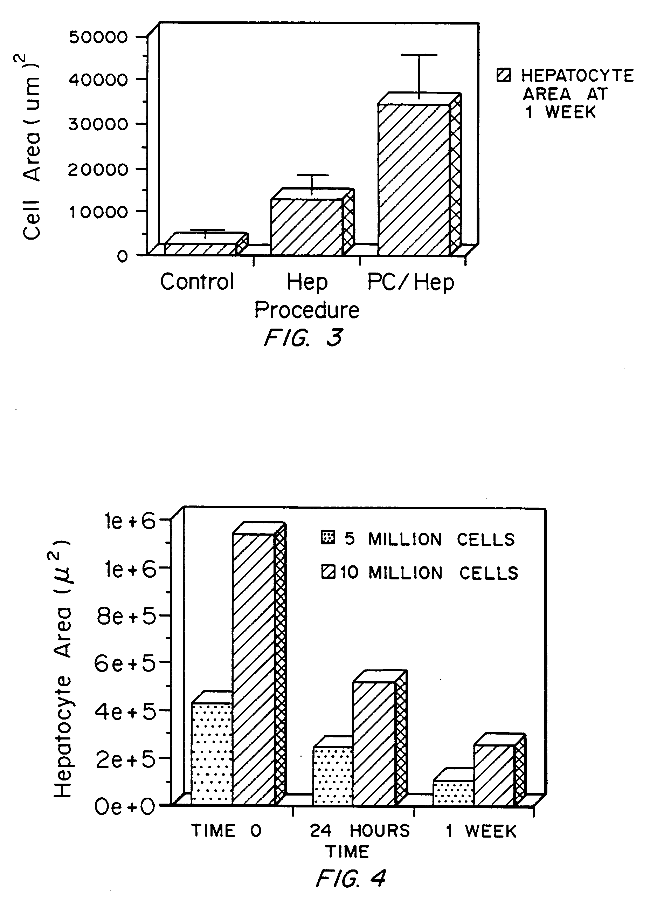

The survival of hepatocytes was determined over a three day time course comparing cell areas of 10-14 fields per section and four sections per sponge. This allowed comparison of cell survival of hepatocytes injected into a similar group of rats from the same hepatocyte isolation. The results are shown in Figure 4.

In the first group of animals, morphometry revealed an average viable cell area of 3153 ± 645 (S.E.M.at time 0 following injection). At three days, the average viable cell area was 1960 ± 567.

These cell areas correlated to an average cell survival of 62% from day 0 to day 3. Calculating for cell number based on a determined average cell size and sponge volume, 3.6 x 10⁶ viable hepatocytes are present at day 3.

In a second group of animals, cell survival at time 0 after injection revealed a cell area of 1089 ± 334 with cell area at 24 hours of 1175 ± 445. This represents complete survival over this period within the error of the method.

Total number of viable cells was 1.8 and 1.9 x 10⁶ cells per sponge, respectively, in these implants.

### RNA Isolation from Polymer Implants

PGA and Ivalon sponge samples were compared for albumin mRNA levels. In the PGA experiment, four polymer pieces were combined for each timepoint, whereas duplicate sponge samples were processed for each timepoint in the Ivalon experiment. Quantitation of slot blot analysis demonstrated a 33-fold, or 97%, decrease in albumin mRNA in hepatocytes on PGA polymer between 0 and 24 hours, consistent with previous results of Northern blot analysis. In contrast, Ivalon sponge samples exhibit a 1.6-fold (36%) decrease at 24 hours. Furthermore, the total amount of Ivalon RNA obtained is similar from 0 to 24 hours, whereas this also dropped dramatically on PGA polymer. These results indicate that hepatocytes maintain the majority of their function *in vivo* at the level of albumin gene expression on Ivalon sponge, but not on PGA polymer.

### Example 5: Effect of hepatotrophic stimulation on graft survival using prevascularized matrices.

These results indicate that prevascularization of a sponge model of hepatocyte implantation significantly improves cell survival in the first 24 hours after implantation. Further increases in survival can be obtained by using portacaval shunting and hepatectomy, and addition of 0₂ directly to implanted cells using a temporary implanted tissue perfusion chamber.

### Methods

### Polymer Implantation

Inbred Lewis rats, 250-350 g (Charles River), were anaesthetized with methoxyflurane. A midline incision was made and the mesentery laid out on a sterile gauze. Ivalon™ (Unipoint Industries) foam discs with a centrally placed silastic injection catheter were fixed into a mesenteric envelope. The injection catheters were led to a distant subcutaneous site. The mesentery with polymer was returned to the abdominal cavity and the incision closed. Animals received a single dose of kefzol 100 at mg/kg.

### Portacaval Shunt

Immediately prior to polymer implantation portacaval shunts were created. The pancreaticoduodenal vein was ligated and transected. The portal vein was mobilized from liver hilum to splenic vein with care taken to avoid the hepatic artery. The vena cava was mobilized posteromedially from the left renal vein to the inferior edge of the liver. At this point the portal vein was ligated at the liver hilum and a non-crushing clamp applied at the level of the splenic vein. A partially occluding clamp was applied to the anteromedial surface of the vena cava. A venotomy was created and end to side portacaval shunt constructed with running 8-0 Prolene*TM* (Ethicon) suture. With adequate flow established, implants were placed as described above.

### Hepatocyte Isolation

A modified Seglan technique [Aiken, 1990 #13], was used for hepatocyte isolation. Following adequate anaesthesia with methoxyflurane the vena cava was cannulated and the liver perfused retrograde with Ca⁺⁺ free saline buffer followed by .05% collagenase D (Boeringer Mannheim) saline buffer with .05 M CaCl. Perfusion was carried out at 39°C. Once hepatocyte dissociation was adequate, the liver was excised and gentle dissociation in Williams E medium (GIBCO) carried out. Viability was assessed using trypan blue nuclear exclusion as the criteria for viability.

Hepatocytes were then further purified using 87% Percoll centrifugation for density separation. The hepatocyte fraction was then resuspended in Williams E medium at 2 x 10⁷ cells/cc. The entire *ex vivo* isolation was carried out at 4°C.

### Hepatocyte Implantation and Hepatectomy

Based on initial studies with hepatocyte injection in prevascularized Ivalon, the polymers were prevascularized for 5 days. This provided optimal tissue and vascular ingrowth for engraftment. Animals were given light metaphane anaesthesia, the injection catheters accessed and hepatocytes injected. 1 x 10⁷ cells were injected (.5 cc) per sponge.

Partial hepatectomy was performed at this point in selected animals with and without PC shunts. Standard 70% hepatectomy was performed.

### Evaluation of Implants

Cell polymer constructs were harvested one week after cell injection. They were fixed, sectioned and stained with H&E. Quantification was carried out using a Model 3000 Image analyzer and computer assisted morphometric analysis. Each device was sectioned at four locations in a consistent fashion. Hepatocyte area was quantified along four cross sections of each of the four histologic sections. This provided a consistent means of assessment. Statistical significance was assessed using analysis of variance.

### Results

Three experimental groups were evaluated. A control group (I) undergoing neither hepatectomy nor portacaval shunt (n=6); a second group (II) which underwent 70% hepatectomy alone (n=6); and the final group (III) which underwent portacaval shunt and 70% hepatectomy (n=6). The cross-sectional hepatocyte area per sponge was 2,643 µ² (SEM ± 1588) for the controls and 12,809 µ² (SEM ± 4074) in animals undergoing hepatectomy alone. For the animals receiving maximal hepatotrophic stimulation with PC shunt and 70% hepatectomy, hepatocyte area reached 34,372 µ² (SEM ± 9752). This is graphically depicted in Figure 3.

This increased engraftment in the animals with PC shunt and hepatectomy translates to a twelve fold increase over the controls and almost three fold increase over hepatectomy alone.

Histologic evaluation also revealed significant morphologic differences between the groups which can be summarized as follows. Control animals with no hepatotrophic stimulation only demonstrated engraftment at the outer edge of the polymer near the interface with the mesentery. Based on prior work, this was a progressive phenomenon with cells initially engrafting throughout the interstices of the polymer but with loss of engraftment occurring centrally in the device. The cells also engrafted in 2-3 cell layer laminates. It was noted that, with the addition of partial hepatectomy, cells engrafted with an acinar arrangement in islands 5-6 cell layers thick. Even more striking was the morphology produced with the addition of portacaval shunt. Large aggregates of cells could be demonstrated throughout the polymer. These cells had a much healthier appearance. As well as an acinar arrangement, the cells arranged into laminae with the cord-like appearance of native liver. Another interesting feature was the presence of tubular duct-like structures. Because these structures looked so similar to biliary ducts, morphometric assessment was carried out. The intralobular ducts of native liver and heterotopic grafts were studied as controls for these structures. The histologic appearance of the hepatocellular structures were remarkably similar to those from heterotopic grafts. Morphometric quantification revealed strikingly similar sizes of the ducts from the implants (745 µ² ± 47) and the transplants (814 µ² ± 40); the native liver (1360 µ² ± 97) had ducts which were significantly larger (p<.001).

Although this invention has been described with reference to specific embodiments, variations and modifications of the method and means for constructing artificial organs by culturing cells on matrices having maximized surface area and exposure to the surrounding nutrient-containing environment will be apparent to those skilled in the art. Such modifications and variations are intended to come within the scope of the appended claims.

## Claims

1. A porous, pliable, non-toxic matrix, the matrix being resistant to compression or tension within the body and suitable for introduction of cells into the matrix and for vascular and connective tissue ingrowth to produce a highly vascularized site, in combination with means for introduction of cells into the matrix following implantation into a patient.

2. The matrix of claim 1 further comprising compounds selected from the group consisting of nutrients, growth factors, cofactors, compounds stimulating angiogenesis, immunomodulators, inhibitors of inflammation, regression factors, factors stimulating differentiation, biologically active molecules stimulating lymphatic network ingrowth, factors enhancing nerve growth, drugs and combinations thereof.

3. The matrix of claim 1 wherein the matrix is configured to provide separate areas of attachment for cells of different origin.

4. The matrix of claim 1 wherein the means for introduction are channels molded into the matrix.

5. The matrix of claim 1 wherein the means for introduction are a catheter.

6. The matrix of claim 1 wherein the pore size of the matrix is between approximately 100 and 300 µm and allows vascular ingrowth and the introduction of cells into the matrix without damage to the cells or patient.

7. The matrix of claim 1 wherein the matrix is formed of a biodegradable polymer selected from the group consisting of polyahydride, polyorthoester, polyglycolic acid, polylactic acid, copolymers and blends thereof and collagen as the matrix material.

8. The matrix of claim 1 wherein the matrix is formed of a non-degradable polymer selected from the group consisting of ethylene vinyl acetate, derivatives of polyvinyl alcohol, polytetrafluoroethylene nylon, and silicon.

9. The matrix of claim 1 further comprising cells selected from the group consisting of bile duct cells, parathyroid cells, thyroid cells, cells of the adrenal-hypothalamic-pituitary axis, heart muscle cells, kidney epithelial cells, kidney tubular cells, kidney basement membrane cells, nerve cells, blood vessel cells, intestinal cells, cells forming bone and cartilage, smooth and skeletal muscle.

10. The matrix of claim 1 further comprising dissociated hepatic cells.

11. The matrix of claim 1 formed of a derivative of polyvinyl alcohol.

12. The use of a matrix according to any one of Claims 1 to 11 in the manufacture of a device for controlled cellular implantation for use in a method of surgery comprising implanting said matrix and waiting until between 10% and 90% of the matrix is vascularized and then introducing viable cells into the vascularized matrix.

13. The use of claim 12 wherein the implantation site is selected from the group consisting of the mesentery, subcutaneous tissue, subfascia, and supraperitoneal.

14. The use of claim 12 wherein the method of surgery further comprises performing a portacaval shunt on the patient.

15. The use of claim 12 wherein the matrix contains means for introducing cells into the matrix at the time of implantation.

16. The use of claim 15 wherein the matrix contains distribution channels for introduction of the cells.

17. The use of claim 15 wherein the matrix contains catheters for introduction of the cells.

18. The use of claim 12 wherein the cells are hepatocytes.

19. The use of claim 12 further comprising selecting the cells from the group consisting of parathyroid cells, thyroid cells, cells of the adrenal-hypothalamic-pituitary axis, nerve cells, cells forming bone and cartilage, and cells forming smooth and skeletal muscle.

## Patentansprüche

1. Poröse, biegsame, nicht-toxische Matrix, die innerhalb des Körpers gegen Druck oder Zug beständig ist und sich für die Einführung von Zellen (in die Matrix) und für ein Gefäß- und Bindegewebeeinwachsen, um eine gefäßreiche Stelle zu bilden,
in Kombination mit Mitteln zum Einführen von Zellen in die Matrix nach der Implantation in einen Patienten eignet.

2. Matrix nach Anspruch 1, zusätzlich umfassend Verbindungen, ausgewählt aus der Gruppe Nährstoffe, Wachstumsfaktoren, Cofaktoren, eine Gefäßbildung stimulierende Verbindungen, Immunmodulatoren, entzündungshemmende Mittel, Regressionsfaktoren, eine Differenzierung stimulierende Faktoren, biologisch aktive Moleküle, die das Einwachsen eines lymphatischen Netzwerks stimulieren, Faktoren, die ein Nervenwachstums begünstigen, Arzneimittel und Kombinationen hiervon.

3. Matrix nach Anspruch 1, die derart ausgestaltet ist, daß zum Haftenbleiben für Zellen unterschiedlicher Herkunft getrennte Bereiche zur Verfügung gestellt werden.

4. Matrix nach Anspruch 1, wobei es sich bei den Mitteln zum Einführen (von Zellen) um in der Matrix ausgeformte Kanäle handelt.

5. Matrix nach Anspruch 1, wobei die Mittel zum Einführen (von Zellen) aus einem Katheter bestehen.

6. Matrix nach Anspruch 1, wobei die Porengröße der Matrix zwischen etwa 100 und 300 µm liegt und ohne Schädigung der Zellen oder des Patienten ein Gefäßeinwachsen und das Einführen von Zellen in die Matrix gestattet.

7. Matrix nach Anspruch 1, welche aus einem biologisch abbaubaren Polymer, ausgewählt aus der Gruppe Polyanhydrid, Polyorthoester, Polyglykolsäure, Polymilchsäure, Copolymere und Mischungen derselben und Collagen als Matrixwerkstoff, gebildet ist.

8. Matrix nach Anspruch 1, die aus einem nicht-abbaubaren Polymer, ausgewählt aus der Gruppe Ethylenvinylacetat, Derivate von Polyvinylalkohol, Polytetrafluorethylen, Nylon und Siliciumpolymere, gebildet ist.

9. Matrix nach Anspruch 1, welche zusätzlich Zellen, ausgewählt aus der Gruppe Gallengangzellen, Nebenschilddrüsenzellen, Schilddrüsenzellen, Zellen der Nebennieren-Hypothalamus-Hypophyse-Achse, Herzmuskelzellen, Nierenepithelzellen, Nierenröhrenzellen, Nierenbasalmembranzellen, Nervenzellen, Blutgefäßzellen, Darmzellen und Zellen zur Bildung von Knochen und Knorpel, glatter Muskeln und Skelettmuskeln, umfaßt.

10. Matrix nach Anspruch 1, die zusätzlich dissoziierte Leberzellen enthält.

11. Matrix nach Anspruch 1, gebildet aus einem Polyvinylalkoholderivat.

12. Verwendung einer Matrix nach einem der Ansprüche 1 bis 11 bei der Herstellung einer Vorrichtung zur gesteuerten Zellenimplantation zur Verwendung im Rahmen eines chirurgischen Verfahrens, umfassend die Implantation der Matrix und Abwarten, bis in 10-90% der Matrix eine Gefäßbildung stattgefunden hat, und anschließendes Einführen lebensfähiger Zellen in die Matrix, in der die Gefäßbildung stattgefunden hat.

13. Verwendung nach Anspruch 12, wobei die Implantationsstelle aus der Gruppe Mesenterium, subkutanes Gewebe, Subfascia und supraperitoneal ausgewählt ist.

14. Verwendung nach Anspruch 12, wobei im Rahmen des chirurgischen Verfahrens zusätzlich am Patienten ein portacavaler Shunt gesetzt wird.

15. Verwendung nach Anspruch 12, wobei die Matrix Mittel zum Einführen von Zellen in die Matrix zum Zeitpunkt der Implantation enthält.

16. Verwendung nach Anspruch 15, wobei die Matrix Verteilungskanäle zum Einführen der Zellen enthält.

17. Verwendung nach Anspruch 15, wobei die Matrix Katheter zum Einführen von Zellen enthält.

18. Verwendung nach Anspruch 12, wobei es sich bei den Zellen um Hepatozyten handelt.

19. Verwendung nach Anspruch 12, wobei zusätzlich die Zellen aus der Gruppe Nebenschilddrüsenzellen, Schilddrüsenzellen, Zellen der Nebennieren-Hypothalamus-Hypophyse-Achse, Nervenzellen und Zellen zur Bildung von Knochen und Knorpel, glatter Muskeln und Skelettmuskeln ausgewählt werden.

## Revendications

1. Matière de support poreuse, flexible, non toxique, la matière de support étant résistante à la compression ou à la tension dans l'organisme et appropriée à l'introduction de cellules dans la matière de support et à la croissance de vaisseaux et de tissu conjonctif, pour la production d'un site hautement vascularisé, en association avec des moyens pour l'introduction de cellules dans la matière de support, après implantation sur un patient.

2. Matière de support selon la revendication 1, comprenant des composés choisis parmi des nutriments, des facteurs de croissance, des cofacteurs, des composés stimulant l'angiogénèse des immunomodulateurs, des anti-inflammatoires, des facteurs de régression, des facteurs stimulant la différentiation, des molécules biologiquement actives stimulant la croissance du système lymphatique, des facteurs stimulant la croissance des neurones, des médicaments et des associations de ceux-ci.

3. Matière de support selon la revendication 1, dans laquelle la matière de support est configurée de manière à procurer des zones d'attachement distinctes pour des cellules d'origine différente.

4. Matière de support selon la revendication 1, dans laquelle les moyens d'introduction sont des canaux moulés dans la matière de support.

5. Matière de support selon la revendication 1, dans laquelle les moyens d'introduction consistent en un cathéter.

6. Matière de support selon la revendication 1, dans laquelle la taille de pores de la matière de support est comprise entre environ 100 et 300 µm et permet la croissance vasculaire et l'introduction de cellules dans la matière de support sans dommages pour les cellules ni pour le patient.

7. Matière de support selon la revendication 1, dans laquelle la matière de support est formée d'un polymère biodégradable choisi parmi un polyanhydride, un polyorthoester, un poly(acide glycolique), un poly(acide lactique), des copolymères et mélanges de ceux-ci et le collagène en tant que matériau de la matière de support.

8. Matière de support selon la revendication 1, dans laquelle la matière de support est formée d'un polymère non dégradable choisi parmi l'éthylène-acétate de vinyle, des dérivés du poly(alcool vinylique), le polytétrafluoroéthylène, le Nylon et les silicones.

9. Matière de support selon la revendication 1, comprenant en outre des cellules choisies dans le groupe constitué par les cellules du canal cholédoque, les cellules parathyroïdiennes, les cellules thyroïdiennes, les cellules de l'axe surrénal-hypothalamique-hypophysaire, les cellules du muscle cardiaque, les cellules épithéliales du rein, les cellules tubulaires du rein, les cellules de la membrane basale du rein, les cellules nerveuses, les cellules des vaisseaux sanguins, les cellules intestinales, les cellules formatrices de l'os et du cartilage, les cellules formatrices des muscles lisses et des muscles squelettiques.

10. Matière de support selon la revendication 1, comprenant en outre des cellules hépatiques dissociées.

11. Matière de support selon la revendication 1, formée d'un dérivé de poly(alcool vinylique).

12. Utilisation d'une matière de support selon l'une quelconque des revendications 1 à 11, dans la fabrication d'un dispositif pour implantation cellulaire contrôlée, destiné à être utilisé dans un procédé chirurgical comprenant l'implantation de ladite matière de support et l'attente jusqu'à ce qu'entre 10 et 90 % de la matière de support soit vascularisés, et ensuite l'introduction de cellules viables dans la matière de support vascularisée.

13. Utilisation selon la revendication 12, dans laquelle le site d'implantation est choisi dans le groupe constitué par le mésentère, le tissu sous-cutané, le site aponévrotique et le site supra-péritonéal.

14. Utilisation selon la revendication 12, dans laquelle le procédé chirurgical comprend en outre la réalisation d'une anastomose portocave sur le patient.

15. Utilisation selon la revendication 12, dans laquelle la matière de support contient des moyens pour l'introduction de cellules dans la matière de support lors de l'implantation.

16. Utilisation selon la revendication 15, dans laquelle la matière de support contient des canaux de distribution pour l'introduction des cellules.

17. Utilisation selon la revendication 15, dans laquelle la matière de support contient des cathéters pour l'introduction des cellules.

18. Utilisation selon la revendication 12, dans laquelle les cellules sont des hépatocytes.

19. Utilisation selon la revendication 12, comprenant en outre le choix des cellules dans le groupe constitué par les cellules parathyroïdiennes, les cellules thyroïdiennes, les cellules de l'axe surrénal-hypothalamique-hypophysaire, les cellules nerveuses, les cellules formatrices de l'os et du cartilage et les cellules formatrices des muscles lisses et des muscles squelettiques.
